# FASCICULE DE BREVET EUROPEEN

(11) **EP 2 541 782 B1**
(45) Date de publication et mention de la délivrance du brevet: **02.04.2014**
(21) Numéro de dépôt: 12169928.4
(22) Date de dépôt: 30.05.2012
(51) Int. Cl.: H04B 1/22, A61N 1/372

(54) **Circuit récepteur de télémétrie RF pour implant médical actif**
RF-Telemetrie-Empfangsschaltkreis für aktives medizinisches Implantat
RF telemetry receiver circuit for active medical implant

(30) Priorité: 28.06.2011 FR 1155759
(43) Date de publication de la demande: 02.01.2013
(73) Titulaire: Sorin CRM SAS, 92140 Clamart (FR)
(72) Inventeur: Makdissi, Alaa, 75011 Paris (FR)
(74) Mandataire: Dupuis-Latour, Dominique

(56) Documents cités:
- DE-A1- 4 440 113
- DE-A1- 10 209 855
- DE-A1- 19 646 746
- US-A- 5 929 779

## Description

L'invention concerne les "dispositifs médicaux actifs" tels que définis par la directive 93/42/CE du 14 juin 1993 du Conseil des communautés européennes, et notamment les implants de type "dispositifs médicaux implantables actifs" tels que définis par la directive du Conseil 90/385/CEE du 20 juin 1990.

Cette définition inclut en particulier les appareils chargés de surveiller l'activité cardiaque et de générer des impulsions de stimulation, de resyn-chronisation, de défibrillation et/ou de cardioversion en cas de trouble du rythme détecté par l'appareil. Elle inclut aussi les appareils neurologiques, les pompes de diffusion de substances médicales, les implants cochléaires, les capteurs biologiques implantés, etc., ainsi que les dispositifs de mesure de pH ou encore d'impédance intracorporelle (telle que mesure d'impédance transpulmonaire ou d'impédance intracardiaque). On notera également que, si l'invention s'applique de manière particulièrement avantageuse aux appareils implantés tels que stimulateurs, cardioverteurs ou défibrillateurs, elle peut tout aussi bien être mise en oeuvre avec des dispositifs médicaux non implantés, par exemple des enregistreurs de données comme les appareils Holter externes destinés à la surveillance et à l'enregistrement en ambulatoire de certains paramètres physiologiques tels que par exemple l'activité cardiaque.

Ces dispositifs médicaux actifs sont conçus pour permettre un échange bidirectionnel de données avec un "programmateur", qui est un dispositif externe permettant de vérifier le paramétrage de l'implant, de lire des informations enregistrées par celui-ci ou d'y inscrire des informations, ou encore de mettre à jour le logiciel interne de pilotage de l'implant.

Il a par ailleurs été proposé des techniques de suivi à domicile (*home monitoring*) des patients, mettant en oeuvre un appareil d'interrogation à distance disposé près du porteur de l'implant, qui est activé à intervalles périodiques, par exemple quotidiennement, pour télécharger les données recueillies par l'implant et les transmettre pour analyse à un site distant de télésurveillance.

L'échange de données entre l'implant et le programmateur ou l'appareil de *home monitoring* (ci-après "dispositif externe") est effectué par télémétrie, c'est-à-dire par une technique de transmission à distance d'informations, sans contact galvanique.

Jusqu'à présent, la télémétrie était le plus souvent opérée par couplage inductif entre des bobines du dispositif implanté et du programmateur, technique connue sous le nom de "procédé par induction". Cette technique présente cependant l'inconvénient, en raison de la très faible portée d'un tel couplage, de nécessiter l'utilisation d'une "tête de télémétrie" reliée au programmateur et contenant une bobine qu'un opérateur place au voisinage du site où est implanté le dispositif.

Il a été récemment proposé de mettre en oeuvre une autre technique de couplage non galvanique, utilisant les deux composantes d'une onde électromagnétique produite par des circuits émetteurs/récepteurs opérant dans le domaine des radiofréquences (RF), typiquement des fréquences de l'ordre de plusieurs centaines de mégahertz.

Cette technique, dite de "télémétrie RF" permet de programmer ou interroger des implants à des distances supérieures à 3 m, et autorise donc l'échange d'informations sans manipulation d'une tête de télémétrie, voire même sans intervention d'un opérateur externe.

Un dispositif médical actif comprenant de tels moyens de télémétrie RF est par exemple décrit dans le EP 1 862 195 A1 (ELA Medical).

Le DE 196 46 746 A1 décrit en détail une technique de télémétrie RF pour la transmission de données d'électrogramme (EGM) recueillies par un implant cardiaque. Cette transmission met en oeuvre une double modulation du signal, à la fois d'amplitude et de fréquence.

La double modulation d'un signal est une technique en elle-même bien connue. Le DE 102 09 855 A1 en donne un autre exemple, pour un système de contrôle d'accès à un véhicule automobile par télécommande codée RF.

La consommation des circuits RF est un aspect crucial de cette technique, surtout en ce qui concerne un implant.

En effet, les implants utilisent comme source d'énergie une batterie dont la capacité est limitée (de l'ordre de 1 Ah). Sans télémétrie, la consommation moyenne d'un implant est de l'ordre de 20 µW, ce qui procure une autonomie de l'ordre d'une dizaine d'années. En revanche, lors de l'activation d'une fonction de télémétrie RF un implant consomme typiquement, avec les technologies actuelles, une dizaine de milliwatts, soit 500 à 1000 fois plus que sa consommation moyenne pour les fonctions usuelles de détection et de stimulation cardiaque.

D'autre part, on sait que l'environnement qui entoure le patient présente de plus en plus des interférences radiofréquence susceptibles de réveiller à tort le circuit de télémétrie, et décharger ainsi inutilement la batterie de l'implant.

Une utilisation occasionnelle de la télémétrie RF une fois tous les trois ou six mois, par exemple à l'occasion de visites chez le cardiologue, ne dégrade pas notablement la durée de vie de l'implant. En revanche, une utilisation quotidienne pour communiquer avec un appareil de *home monitoring* peut réduire notablement la durée de vie de l'implant.

En effet, si l'on affecte par exemple le quart de la capacité de la batterie de l'implant aux fonctions de télémétrie RF, cette capacité ne procurera que 50 heures de communication RF pendant toute la durée de vie de l'implant (10 ans), soit une utilisation quotidienne de moins de 50 secondes.

Sachant que les systèmes de télémétrie actuels utilisent des canaux de communication limités à 300 kHz avec un débit de 100 à 200 kbps (kilo-bits par seconde) environ, et que les signaux EGM sont échantillonnés sur 10 bits et 500 fois par seconde, une durée de transmission de 50 secondes ne permet la transmission à l'appareil externe (en tenant compte de la surcharge introduite par le protocole de communication) que de 8 minutes au plus d'enregistrement de deux voies de signaux EGM.

Il existe donc un besoin d'un système de télémétrie RF utilisable à distance d'une façon intense mais de façon économe sur le plan énergétique, donc sans pénaliser la durée de vie de l'implant.

Tel est le problème de l'invention.

Le point de départ de l'invention réside dans la mise en oeuvre dans l'implant de circuits de communication dont la consommation et la complexité sont minimales, quitte à augmenter la complexité et la consommation des circuits des dispositifs externes. En effet, ces derniers fonctionnent avec une source d'énergie à capacité beaucoup plus grande que celle de la batterie d'un implant, et qui est en outre aisément remplaçable ou rechargeable.

En particulier, le signal émis par le dispositif externe sera choisi avec une structure de modulation permettant de rendre le circuit de réception dans l'implant le plus simple possible et de consommation très faible, la complexité et la consommation supplémentaire pour générer ce signal spécifiquement modulé étant à la charge du dispositif externe.

Très avantageusement, l'optimisation portera également sur le choix des bandes de fréquences utilisées pour la transmission des signaux RF.

Par ailleurs, aussi bien en ce qui concerne le mode de modulation que les canaux de la bande de fréquences choisie, rien n'empêche de prévoir une configuration non symétrique entre les deux sens de communication (le sens "descendant" du dispositif externe vers l'implant et le sens "montant" de l'implant vers le dispositif externe).

On verra plus loin que ces divers choix permettent, dans le sens descendant, de réduire la consommation du système de télémétrie d'un à deux ordres de grandeur (facteur d'amélioration de x10 à x100) par rapport aux systèmes actuels, permettant ainsi de préserver dans une très large mesure la durée de vie de l'implant même en cas d'utilisation intensive de la télémétrie RF.

On verra également que la structure (modulation) du signal RF transmis du dispositif externe vers l'implant, dans le sens descendant, permet de réduire largement les interférences RF, évitant ainsi de dégrader la consommation et donc l'autonomie de l'implant par des faux réveils du circuit de réception.

La chaîne de réception selon l'invention est une chaîne "semi-passive", c'est-à-dire où les composants consommateurs d'énergie sont réduits au strict minimum. En particulier, cette chaîne de réception ne comporte ni oscillateur local ni mélangeur, à la différence des circuits conventionnels de réception à démodulateur de type hétérodyne. Le circuit de réception peut même être dépourvu d'amplificateur de type LNA (*Low Noise Ampli fier*, amplificateur à faible bruit).

On verra enfin que cette chaîne de réception est non seulement "semi-passive" mais également particulièrement sélective, ce qui la rend utilisable notamment dans des bandes de fréquences partagées par plusieurs utilisateurs, permettant ainsi le choix d'une bande éventuellement plus appropriée que celle(s) habituellement dévolue(s) à la transmission RF dans le domaine des implants médicaux.

Plus précisément, le circuit récepteur de télémétrie RF selon l'invention comprend, de façon en elle-même connue, par exemple d'après le document DE 196 46 746 A1 précité : un circuit récepteur de télémétrie RF pour implant médical actif, avec une chaîne de réception comprenant successivement : une antenne de réception d'un signal RF ; un étage amont de filtrage passe-bande et d'adaptation d'impédance du signal capté par l'antenne ; un étage détecteur d'enveloppe du signal délivré par l'étage amont de filtrage ; et un étage de démodulation numérique du signal délivré par l'étage détecteur d'enveloppe. L'étage détecteur d'enveloppe est un étage apte à démoduler une forme d'onde d'un signal RF à double modulation, présentant : une première modulation, d'une première porteuse par un signal binaire en bande de base à transmettre, pour donner un premier signal modulé, et une seconde modulation, pour donner un second signal modulé à transmettre.

De façon caractéristique, la seconde modulation est une modulation d'une seconde porteuse par le premier signal modulé, la première porteuse étant à basse fréquence et la seconde porteuse étant à haute fréquence. L'étage amont de filtrage est centré sur la fréquence de la porteuse haute fréquence. L'étage détecteur d'enveloppe est un étage de détection non hétérodyne, dépourvu d'oscillateur local et de mélangeur, et comprend : un premier circuit de détection non cohérente à diode, recevant en entrée le signal délivré par l'étage amont de filtrage ; un filtre passe-bande actif, recevant en entrée le signal délivré par le premier circuit de détection non cohérente à diode, ce filtre passe-bande actif étant centré sur une fréquence double de celle de la porteuse basse fréquence et présentant une largeur de bande double de celle de la bande de base ; et un second circuit de détection non cohérente à diode, recevant en entrée le signal délivré par le filtre passe-bande actif et délivrant en sortie un signal en bande de base appliqué à l'étage de démodulation numérique.

Selon diverses caractéristiques particulières préférentielles :
- le premier circuit de détection non cohérente à diode comprend une source de courant de polarisation de diode ;
- le premier circuit de détection non cohérente à diode comprend un détecteur à deux diodes configurées en doubleur de tension ;
- la fréquence de la porteuse haute fréquence est comprise dans la bande ISM 2,4 GHz ou la bande RFID 900 MHz ;
- la fréquence de la porteuse basse fréquence est comprise entre 25 et 500 kHz ;
- la fréquence du signal en bande de base est comprise entre 5 et 100 kbps.
- le gain du filtre passe-bande actif est compris entre 30 et 50 dB.

On va maintenant décrire un exemple de mise en oeuvre du dispositif de l'invention, en référence aux dessins annexés où les mêmes références numériques désignent d'une figure à l'autre des éléments identiques ou fonctionnellement semblables.
La Figure 1 est une vue schématique d'un ensemble constitué d'un implant et d'un dispositif externe, illustrant les blocs fonctionnels mis en jeu dans l'échange de données par télémétrie RF.
La Figure 2 est une représentation schématique des principaux blocs fonctionnels de la partie d'émission du dispositif externe.
La Figure 3 illustre de façon schématique les principales fonctions mises en oeuvre dans un circuit de réception de type hétérodyne selon l'état de la technique.
La Figure 4 illustre de façon schématique les principales fonctions mises en oeuvre dans un circuit de réception à détection non cohérente à diode Schottky selon l'état de la technique.
La Figure 5 présente une série de chronogrammes de signaux relevés en divers points du circuit émetteur du dispositif externe illustré Figure 3.
La Figure 6 est homologue de la Figure 5, montrant la répartition des différents signaux en question, considérés dans le domaine fréquentiel.
La Figure 7 illustre de façon schématique les principales fonctions mises en oeuvre par la chaîne de réception semi-passive de l'implant, selon l'invention.
La Figure 8 illustre une variante du circuit de la Figure 7, avec courant de polarisation et doubleur de tension.
La Figure 9 présente une série de chronogrammes de signaux relevés en divers points de la chaîne de réception de la Figure 7.

On va maintenant décrire un exemple de réalisation de l'invention.

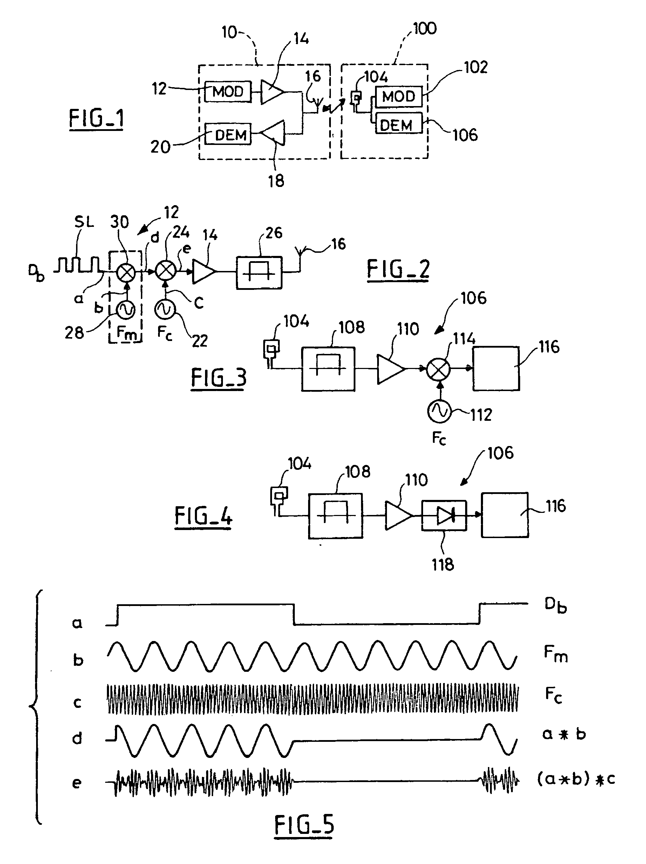

Sur la Figure 1 on a illustré de façon schématique les différents circuits d'émission/réception d'un dispositif externe 10 communiquant avec un implant 100.

Le dispositif externe 10 est équipé d'un modulateur 12 délivrant un signal à un amplificateur de puissance 14 alimentant une antenne d'émission 16. Pour la partie réception, les signaux recueillis par l'antenne 16 sont amplifiés par un amplificateur faible bruit LNA 18 alimentant un démodulateur 20.

En ce qui concerne l'implant 100, celui-ci comprend un circuit modulateur 102 alimentant une antenne 104 pour transmission des données au dispositif externe 10 (transmission RF dans le sens montant). Dans le sens descendant (sens inverse, du dispositif externe 10 vers l'implant 100), un démodulateur 106 recueille les signaux captés par l'antenne 104 de l'implant.

L'échange de données RF entre le dispositif externe 10 et l'implant 100 peut être asymétrique c'est-à-dire que :
- d'une part, les types de modulation ne sont pas les mêmes dans un sens et dans l'autre (c'est-à-dire que les deux modulateurs 12 et 102 n'opèrent pas selon la même technique, de même que les démodulateurs 20 et 106, par voie de conséquence), et
- dans la bande de fréquences choisie, des canaux différents peuvent être utilisés dans un sens et dans l'autre.

La Figure 2 décrit plus précisément l'étage de modulation 12 du dispositif externe 10. Celui-ci comporte un oscillateur local 22 à fréquence F_{C} appliquée à un mélangeur 24 recevant par ailleurs le signal numérique à transmettre (signal logique SL). Le signal résultant, après passage par l'amplificateur de puissance 14, est appliqué à l'antenne 16 via un filtre passe-bande sélectif 26 permettant de limiter le spectre résiduel du signal en dehors de la bande de transmission considérée.

La Figure 3 illustre de façon schématique les principales fonctions mises en oeuvre dans un circuit de réception de type hétérodyne selon l'état de la technique, pour la réception et la démodulation du signal émis par le dispositif externe 10.

Dans un tel récepteur conventionnel (cf. l'article "*Récepteur radio*" de l'encyclopédie Wikipedia), le signal capté par l'antenne 104 est appliqué à un circuit 108 d'adaptation d'impédance et de filtrage passe-bande, permettant de maximiser le rapport signal/bruit à l'entrée de la chaîne de réception. Le signal résultant est appliqué à un amplificateur à faible bruit LNA 110 qui amplifie le signal reçu dans la bande de fréquences de la porteuse F_{C}. La démodulation est opérée par un oscillateur local 112 à fréquence F_{C} et un mélangeur 114 recevant le signal reçu filtré. Le signal en bande de base obtenu par translation de fréquence est ensuite appliqué à un étage 116 de démodulation numérique permettant de restituer en sortie le signal logique originel transmis depuis le dispositif externe.

On notera que cette structure conventionnelle de chaîne de réception comprend l'amplificateur 110, l'oscillateur local 112 et le mélangeur 114 qui sont des circuits actifs, pouvant représenter jusqu'à 90 % de la consommation globale de la chaîne de réception (l'étage de démodulation numérique 116, qui fonctionne à basse fréquence, consomme peu d'énergie).

La Figure 4 présente une variante de chaîne de réception, également connue (cf. par exemple le US 5 929 779 A, Figure 3), ne comportant pas d'oscillateur local et d'étage mélangeur. Ces éléments sont remplacés par une diode 118 de détection non cohérente, par exemple une diode Schottky, qui est un composant passif.

Le principal composant actif consommateur d'énergie est l'amplificateur 110, ce qui conduit à une consommation deux à trois fois moindre que celle du circuit à détection hétérodyne conventionnel de la Figure 3.

Ce circuit présente cependant un inconvénient tenant au fait que le détecteur à diode 118 opère selon une loi quadratique de conversion en tension du signal RF amplifié. On montre qu'un tel récepteur est pour cette raison un récepteur non sélectif, qui détecte donc en même temps tous les signaux RF présents dans la bande de communication.

Cette absence de sélectivité rend ce type de récepteur inutilisable dans des bandes de fréquences partagées par plusieurs utilisateurs. Il est certes possible d'améliorer la sélectivité par des filtres à bande très étroite, mais qui sont difficilement réalisables dans un implant en raison de leur taille importante.

En tout état de cause, même si l'on réussit à obtenir un récepteur accordé sur un canal unique de communication pour éliminer les interférences des autres canaux de la même bande de communication, la consommation de l'amplificateur 110 reste élevée (quelques milliwatts) et ne permet pas une utilisation intensive de ce récepteur dans un implant cardiaque dont la consommation permanente est, comme on l'a indiqué plus haut, de quelques dizaines de microwatts seulement.

On va maintenant décrire la technique mise en oeuvre par l'invention pour pallier ces différents inconvénients.

L'invention prévoit d'utiliser une double modulation du signal dans le sens descendant (du dispositif externe vers l'implant).

Le dispositif externe est adapté en conséquence : plus précisément, si l'on se réfère à la Figure 2 décrite plus haut, outre la modulation par la porteuse principale à la fréquence F_{C} (oscillateur 22 et mélangeur 24), on prévoit une modulation préalable du signal à une fréquence plus faible Fₘ, par un autre oscillateur local 28 associé à un autre mélangeur 30.

La Figure 5 illustre les chronogrammes des signaux relevés aux différents points a à e de la chaîne de modulation du dispositif externe de la Figure 5.

Le chronogramme a illustre le signal logique SL contenant l'information à transmettre. Ce signal est produit à un débit numérique D_{b} (signal en bande de base) de l'ordre de 5 à 100 kbps. On peut par exemple utiliser une modulation du type NRZ (non retour à zéro) dans laquelle les uns binaires sont représentés par un signal logique d'amplitude V donnée, et les zéros logiques par une amplitude nulle.

Ce signal a en bande de base va être soumis à une double modulation, respectivement par les signaux de porteuse basse fréquence Fₘ et haute fréquence F_{C}. On précisera ici que les termes "basse fréquence" et "haute fréquence" sont des termes relatifs (les deux fréquences sont différentes, et l'une est plus élevée que l'autre), et n'impliquent aucune connotation particulière sur la valeur choisie, dans l'absolu, pour chacune de ces fréquences. Les valeurs numériques préférentielles que l'on donnera ne devront ainsi être considérées que comme des valeurs illustratives, sans caractère limitatif.

La porteuse basse fréquence Fₘ (signal *b*) est avantageusement à une fréquence de l'ordre de cinq à dix fois la fréquence de la bande de base D_{b}, c'est-à-dire une fréquence comprise entre 25 et 500 kHz.

La porteuse haute fréquence F_{C} (signal *c*) correspond à la bande RF choisie, qui est avantageusement la bande 2,4 GHz (plus précisément, la bande comprise entre 2,40 et 2,45 GHz), qui est la banalisée publique ISM (Industriel, Scientifique et Médical).

Cette bande ISM 2,4 GHz est en effet préférée à la bande MICS (*Medical Implant Communication System*) 402-405 MHz généralement utilisée par les dispositifs médicaux, pour les raisons suivantes.

Dans la bande 2,4 GHz, un signal RF transmis depuis un dispositif externe situé à deux mètres du patient jusqu'à un implant situé à quelques centimètres sous la peau de celui-ci subit une atténuation de l'ordre de 70 à 80 dB. Dans la bande MICS, la puissance maximale autorisée n'est que de -16 dBm (25 µW). En revanche, dans la bande ISM la puissance maximale autorisée est de 20 dBm (100 mW), et peut atteindre 30 dBm (1 W) si l'on utilise des techniques de modulation numérique telles que les sauts de fréquence (*Frequency Hopping*)*.*

Il est possible de disposer ainsi d'une puissance bien plus élevée - de l'ordre de 1000 à 10 000 fois supérieure (30 à 40 dB) et ainsi de recueillir une puissance plus importante dans l'implant, avec une moindre amplification du signal.

Ainsi, pour un signal dont la puissance RF émise varie entre 10 dBm et 30 dBm (10 mW et 1 W) et une atténuation de 70 à 80 dB, la puissance du signal RF reçu par l'implant, avant toute amplification, sera comprise entre -40 dBm et -70 dBm (100 mW et 100 pW).

Le chronogramme d représente le signal logique après modulation par la porteuse basse fréquence (produit des signaux *a* et *b*), et le chronogramme e le même signal après la seconde modulation par la porteuse haute fréquence F_{C} (produit des signaux *d* et *c*).

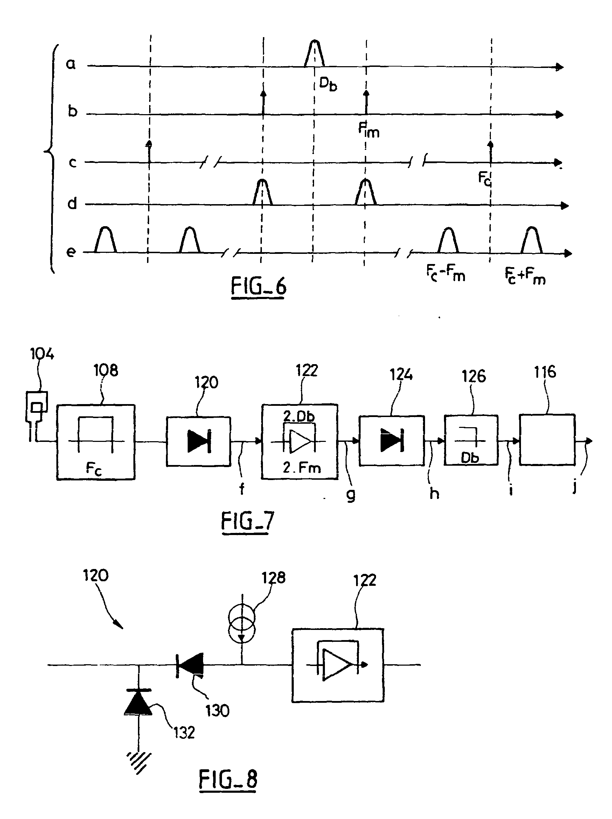

La Figure 6 montre la répartition des différents signaux a à e dans le domaine fréquentiel.

Le spectre du signal a est celui du signal binaire en bande de base D_{b}, centré autour de la fréquence zéro, et occupe la bande de fréquences entre -D_{b} et +D_{b}.

Le spectre du signal *b* est celui de la porteuse basse fréquence, et comprend un pic à la fréquence +Fₘ et un pic à la fréquence -Fₘ.

Le spectre du signal *c* est celui de la porteuse haute fréquence, et comprend un pic à la fréquence +F_{C} et un pic à la fréquence -F_{C}.

Le spectre du signal *d* (après la première modulation) comprend le spectre du signal en bande de base *a*, centré autour de la fréquence +Fₘ. La largeur de bande occupée est égale à 2 D_{b}.

Enfin, le spectre du signal *e* (après la seconde modulation) contient deux fois le spectre du signal en bande de base, avec une sous-bande centrée autour de F_{C}-Fₘ, et une autre autour de F_{C}+Fₘ, les deux sous-bandes étant donc distantes de 2.Fₘ.

La double modulation n'est certes pas efficace spectralement, car on voit que le spectre du signal transmis occupe quatre fois la bande du signal binaire en bande de base.

En revanche, bien que cette double modulation ne soit pas optimale d'un point de vue spectral, on verra par la suite qu'elle permet de réaliser un récepteur semi-passif sélectif dans l'implant.

Cette sélectivité, très importante pour obtenir un récepteur de bonne qualité, exploite le fait que les deux sous-bandes du spectre du signal émis (signal *e*) sont toujours séparées par 2.Fₘ quelle que soit la fréquence de la porteuse haute fréquence F_{c}.

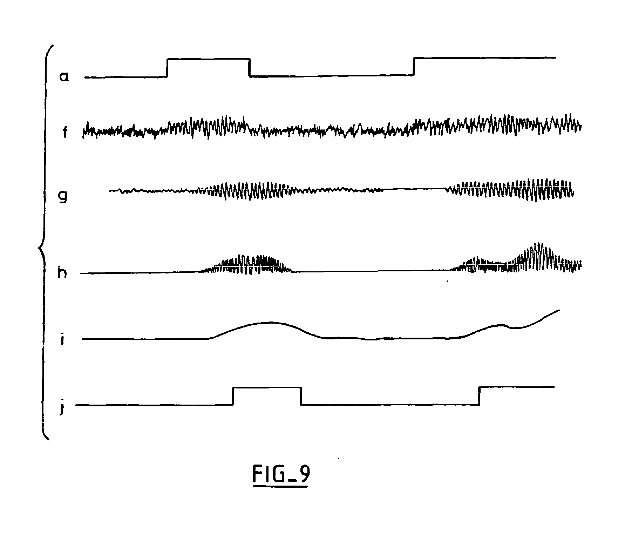

On va maintenant décrire, en référence à la Figure 7, la chaîne de réception semi-passive de l'implant, selon l'invention. La Figure 9 présente une série de chronogrammes de signaux relevés en divers points.

Cette chaîne de réception est spécifiquement adaptée à la double modulation que l'on vient de décrire.

Le signal doublement modulé est capté par l'antenne 104. Ce signal est appliqué à un étage 108 d'adaptation d'impédance et de filtrage passif passe-bande centré sur la fréquence de la porteuse haute fréquence F_{C}, pour atténuer les signaux en dehors de la bande choisie. Le signal ainsi filtré est appliqué à un premier circuit de détection non cohérente 120, comprenant une diode, de préférence une diode Schottky, qui opère une détection d'enveloppe par redressement du signal selon une loi quadratique.

Le signal délivré par ce premier détecteur d'enveloppe 120 (signal *f* sur la Figure 9) est appliqué à un filtre 122 qui, de façon caractéristique, est un filtre passe-bande actif centré autour de la fréquence 2.Fₘ, et ayant une largeur de bande de l'ordre de 2.D_{b}.

Un tel filtre actif est aisément réalisable en technologie CMOS standard, peu consommatrice d'énergie. Ce filtre possède un gain suffisamment élevé pour rendre la détection du signal dans les étages suivants de la chaîne de réception facilement réalisable.

Il est ainsi possible de réaliser un tel filtre actif avec un gain en tension de 100 (40 dB) pour une consommation de l'ordre de 10 pA.

On notera par ailleurs que la double modulation (porteuse basse fréquence et porteuse haute fréquence) utilisée par le circuit de l'invention permet de s'affranchir des limites d'un circuit conventionnel à détection non cohérente à diode de l'art antérieur tel que celui illustré Figure 4.

En effet, dans un tel détecteur opérant sur une fréquence porteuse unique, on obtient seulement en sortie de l'étage de détection une composante continue, et ceci pour toutes les fréquences situées dans la bande utile, c'est-à-dire la bande sélectionnée par le filtre amont 108. Cette composante continue est très corrompue et bruitée, avec pour conséquence une transmission de médiocre qualité du signal numérique du dispositif externe vers l'implant.

La double modulation utilisée par l'invention permet au contraire d'obtenir une transmission beaucoup plus robuste car, du fait de la double modulation, la détection par la diode 120 génère en sortie non plus une composante continue, mais un signal à la fréquence du filtre passe-bande 122. De ce fait, le traitement du signal en aval pour obtenir les informations numériques transmises se fera sur la base de la composante située autour de la fréquence 2.Fₘ, et non d'une composante continue corrompue et bruitée.

On notera par ailleurs que le signal *f* en sortie du premier circuit détecteur d'enveloppe 120 après filtrage de la composante haute fréquence par le filtre 108 présente un rapport signal/bruit de +5 dB, bien inférieur à la sensibilité tangentielle de +8 dB de la diode du circuit 120. En effet, bien que les signaux en sortie d'un détecteur à diode soient très faibles, c'est le niveau du bruit dans ces signaux très faibles qui détermine la sensibilité du récepteur.

Dans le cas présent, compte tenu de la double modulation du signal, il est possible de détecter un niveau de signal très faible en sortie du détecteur à diode 120. La sensibilité de ce détecteur peut être caractérisée par le paramètre TSS de sensibilité tangentielle du signal (*Tangential Signal Sensitivity*), définie comme étant le niveau du signal qui donne un rapport signal/bruit égal à 8 dB à la sortie du détecteur. Cette sensibilité tangentielle est liée à la largeur de bande de l'étage d'amplification en aval du détecteur selon la relation TSS = K√B, K étant une constante et B étant la largeur de bande en sortie. Cette relation montre que la sensibilité du récepteur à diode peut être largement améliorée en réduisant la largeur de bande du signal en sortie, autrement dit en augmentant le temps d'intégration du signal de sortie.

Dans le cas présent, l'amplificateur du filtre actif 122 présente une largeur de bande B=2.D_{b}, comme indiqué plus haut. La constante K liant la bande B à la TSS est de l'ordre de 1,4 x 10⁻¹² dans les détecteurs à diode de la famille *HSMS-285x* de Avago Technologies, Inc. Pour un débit de 5 kbps, cette largeur de bande est 10 kHz, soit une sensibilité TSS de -68,5 dBm ; pour un débit de 100 kbps, la TSS vaudra -62 dBm.

Le chronogramme *g* de la Figure 9 montre le signal obtenu en sortie du filtre passe-bande actif 122.

Ce signal est appliqué à un deuxième circuit détecteur d'enveloppe à diode 124 donnant en sortie un signal (*h* sur la Figure 9) correspondant à la valeur absolue (ou redressée) du signal *g* appliqué en entrée. Ce circuit est suivi par un filtre passe-bas 126 ajusté sur une fréquence de coupure correspondant à la fréquence de la bande de base D_{b}. Le signal résultant est illustré en *i* sur la Figure 9.

Ce dernier signal est appliqué à un étage de démodulation numérique 116, par exemple constitué d'un comparateur à hystérésis combiné à un compteur, qui permet de retrouver (signal *j*) en sortie le signal originel émis par le dispositif externe (signal *a*) avec simplement un décalage temporel correspondant au temps d'intégration des circuits. Le compteur peut fonctionner avec une horloge à faible consommation à fréquence basse, de l'ordre de 5.D_{b} c'est-à-dire de l'ordre de 25 à 500 kHz. La consommation d'un tel circuit de démodulation numérique 116 pourra être de l'ordre de 1 pA.

On notera que la chaîne de réception selon l'invention que l'on vient de décrire ne contient aucun circuit actif fonctionnant dans la bande de fréquences de la porteuse haute fréquence F_{C}, limitant d'autant la consommation du circuit de réception.

La consommation totale de la chaîne de réception illustrée Figure 7 peut ainsi être inférieure à 15 pA, soit 100 à 500 fois moins que les meilleurs récepteurs hétérodynes existant aujourd'hui.

En ce qui concerne la sensibilité de ce circuit de réception, celle-ci est essentiellement fonction du rapport signal/bruit au niveau de l'étage contenant le premier détecteur d'enveloppe 120.

On estime en général que la sensibilité d'un récepteur à détection non cohérente (tel que celui illustré Figure 3) est bien inférieure à celle d'un récepteur hétérodyne (tel que celui illustré Figure 3) : ainsi, les récepteurs à détection d'enveloppe non cohérente dans la bande ISM 2,4 MHz présentent généralement une sensibilité comprise entre -40 et -50 dBm.

Ceci laisserait à penser qu'un tel montage serait difficilement utilisable dans un implant, où la puissance des signaux reçus varie typiquement entre -40 et -70 dBm, selon la puissance d'émission. Le circuit de la présente invention va, précisément, à l'encontre de cette idée généralement admise.

Il est possible d'améliorer encore la sensibilité de la chaîne de réception que l'on vient de décrire.

En particulier, une impédance d'entrée élevée de l'étage amplificateur du filtre actif passe-bande 122 améliore la sensibilité, et permet d'utiliser une porteuse basse-fréquence à une fréquence Fₘ relativement faible.

Une autre manière d'améliorer la sensibilité en réduisant la consommation consiste à utiliser pour l'étage 120 deux diodes 130, 132 montées en doubleur de tension, comme illustré Figure 8. L'utilisation de deux diodes pour double la tension permet d'augmenter le niveau du signal en entrée du filtre actif 122 et réduire ainsi le gain de cet amplificateur, avec une baisse corrélative de sa consommation.

Une autre manière encore d'augmenter la sensibilité consiste, comme illustré également Figure 8, à injecter dans la (les) diode(s) un courant de polarisation de l'ordre de 100 nA à 10 pA au moyen d'un générateur de courant 128. La valeur de ce courant de polarisation est choisie de manière à maximiser la sensibilité en fonction des caractéristiques propres de la diode utilisée, en tenant compte notamment du fait que la sensibilité tangentielle TSS n'est pas linéaire. La polarisation, fixe ou variable en fonction de la puissance reçue, permet ainsi d'optimiser le gain de conversion énergie/tension de la (des) diode(s).

Par rapport à un circuit tel que celui illustré Figure 7 sans courant de polarisation et avec une seule diode, la configuration de la Figure 8 procure un gain de 6 dB, soit une sensibilité de l'ordre de -75 dBm pour un débit D_{b} de 5 kbps.

Bien que cette sensibilité de -75 dBm soit inférieure à celle obtenue avec un récepteur hétérodyne (de l'ordre de -100 dBm pour un débit de 100 kbps), elle est largement suffisante pour obtenir un récepteur de très bonne qualité pour de faibles débits de signaux. En effet, dans le sens descendant (du dispositif externe vers l'implant) le volume des informations à transmettre est relativement limité, dans la mesure où il s'agit principalement de commandes et de paramètres envoyés vers l'implant - à la différence du sens montant, où il s'agit de télécharger depuis l'implant un volume important de données stockées dans la mémoire de celui-ci, par exemple des données EGM recueillies sur une période de 24 heures, voire plusieurs jours.

Par ailleurs, en termes de coût énergétique, la consommation d'un récepteur conventionnel hétérodyne de 10 mW pour un débit de 200 kbps conduit à un coût de 50 nJ/bit, alors que le récepteur de la présente invention consomme moins de 50 µW pour un débit de 100 kbps, soit un coût énergétique de 0,5 nJ/bit, cent fois moindre.

Outre la réduction de la consommation, la simplicité de construction de la chaîne de réception (essentiellement, deux détecteurs à diodes et un amplificateur sélectif basse fréquence) permet une réalisation matérielle très simple, sans aucun composant actif fonctionnant dans la bande des fréquences RF.

Un autre avantage encore du récepteur selon l'invention est sa capacité de prendre en compte un signal dont la fréquence de la porteuse RF (la porteuse haute fréquence F_{c}) varie dans le temps, par exemple en cas de modulation avec des sauts de fréquence pour sécuriser la chaîne de réception ou pour rechercher parmi plusieurs canaux possibles celui qui est le moins bruité et/ou assurera la meilleure transmission : en effet, le premier circuit de détection d'enveloppe 120 est insensible à la fréquence de la porteuse F_{c}, de sorte qu'il n'est nullement nécessaire de régler un quelconque canal de communication côté récepteur.

## Revendications

1. Un circuit récepteur de télémétrie RF pour implant médical actif, avec une chaîne de réception comprenant successivement :
- une antenne (104) de réception d'un signal RF ;
- un étage amont (108) de filtrage passe-bande et d'adaptation d'impédance du signal capté par l'antenne ;
- un étage détecteur d'enveloppe du signal délivré par l'étage amont de filtrage ; et
- un étage (116) de démodulation numérique du signal délivré par l'étage détecteur d'enveloppe,
l'étage détecteur d'enveloppe étant un étage apte à démoduler une forme d'onde d'un signal RF à double modulation, présentant :
- une première modulation, d'une première porteuse (*b*) par un signal binaire (*a*) en bande de base (D_{b}) à transmettre, pour donner un premier signal modulé (*d*), et
- une seconde modulation, pour donner un second signal modulé (*e*) à transmettre,
**caractérisé en ce que** :
- la seconde modulation est une modulation d'une seconde porteuse (*c*) par le premier signal modulé, la première porteuse étant à basse fréquence (Fₘ) et la seconde porteuse étant à haute fréquence (F_{C}) ;
- l'étage amont (108) de filtrage est centré sur la fréquence (F_{C}) de la porteuse haute fréquence ; et
- l'étage détecteur d'enveloppe est un étage de détection non hétérodyne, dépourvu d'oscillateur local et de mélangeur, et comprend :
• un premier circuit (120) de détection non cohérente à diode, recevant en entrée le signal délivré par l'étage amont de filtrage ;
• un filtre passe-bande actif (122), recevant en entrée le signal (*f*) délivré par le premier circuit de détection non cohérente à diode, ce filtre passe-bande actif étant centré sur une fréquence (2.Fₘ) double de celle (Fₘ) de la porteuse basse fréquence et présentant une largeur de bande (2.D_{b}) double de celle de la bande de base (D_{b}) ; et
• un second circuit (124) de détection non cohérente à diode, recevant en entrée le signal (*g*) délivré par le filtre passe-bande actif et délivrant en sortie un signal (*h*) en bande de base appliqué à l'étage de démodulation numérique (116).

2. Le circuit récepteur de la revendication 1, dans lequel le premier circuit de détection non cohérente à diode comprend une source (128) de courant de polarisation de diode.

3. Le circuit récepteur de la revendication 1, dans lequel le premier circuit de détection non cohérente à diode comprend un détecteur à deux diodes (130, 132) configurées en doubleur de tension.

4. Le circuit récepteur de la revendication 1, dans lequel la fréquence (F_{c}) de la porteuse haute fréquence est comprise dans la bande ISM 2,4 GHz ou dans la bande RFID 900 MHz.

5. Le circuit récepteur de la revendication 1, dans lequel la fréquence (Fₘ) de la porteuse basse fréquence est comprise entre 25 et 500 kHz.

6. Le circuit récepteur de la revendication 1, dans lequel la fréquence (D_{b}) du signal en bande de base est comprise entre 5 et 100 kbps.

7. Le circuit récepteur de la revendication 1, dans lequel le gain du filtre passe-bande actif (122) est compris entre 30 et 50 dB.

## Patentansprüche

1. RF-Telemetrie-Empfangsschaltung für ein aktives medizinisches Implantat mit einer Empfangskette, umfassend nacheinander:
- eine Empfangsantenne (104) für ein RF-Signal;
- eine stromaufwärtige Stufe (108) zur Bandpassfilterung und Impedanzanpassung des von der Antenne erfassten Signals;
- eine Stufe zur Hüllenerfassung des von der stromaufwärtigen Filterstufe gelieferten Signals; und
- eine Stufe (116) der digitalen Demodulation des von der Hüllenerfassungsstufe gelieferten Signals,
wobei die Hüllenerfassungsstufe eine Stufe ist, die geeignet ist, eine Wellenform eines RF-Signals mit doppelter Modulation zu modulieren, umfassend:
* eine erste Modulation einer ersten Trägerwelle (*b*) durch ein binäres Signal (a) in ein zu übertragendes Basisband (D_{b}), um ein erstes moduliertes Signal (d) zu ergeben, und
* eine zweite Modulation, um ein zweites zu übertragendes moduliertes Signal (*e*) zu ergeben,
**dadurch gekennzeichnet, dass**:
- die zweite Modulation eine Modulation einer zweiten Trägerwelle (*c*) durch das erste modulierte Signal ist, wobei die erste Trägerwelle eine niedrige Frequenz (Fₘ) und die zweite Trägerwelle eine hohe Frequenz (F_{c}) aufweist;
- die stromaufwärtige Filterstufe (108) auf der Frequenz (F_{c}) der Hochfrequenzträgerwelle zentriert ist; und
- die Hüllenerfassungsstufe eine nicht heterodyne Erfassungsstufe ohne lokalen Oszillator und Mischer ist und umfasst:
* eine erste nicht kohärente Erfassungsschaltung (120) mit Diode, die am Eingang das von der stromaufwärtigen Filterstufe gelieferte Signal empfängt;
* einen aktiven Bandpassfilter (122), der am Eingang das Signal (*f*) empfängt, das von der ersten nicht kohärenten Erfassungsschaltung mit Diode geliefert wird, wobei dieser aktive Bandpassfilter auf einer doppelten Frequenz (2.Fₘ) von jener (Fₘ) der Niederfrequenzträgerwelle zentriert ist und eine doppelte Bandbreite (2.D_{b}) von jener des Basisbandes (D_{b}) aufweist; und
* eine zweite nicht kohärente Erfassungsschaltung (124) mit Diode, die am Eingang das Signal (*g*) empfängt, das von dem aktiven Bandpassfilter geliefert wird, und am Ausgang ein Signal (*h*) im Basisband liefert, das an die digitale Demodulationsstufe (116) angelegt wird.

2. Empfangsschaltung nach Anspruch 1, bei der die erste nicht kohärente Erfassungsschaltung mit Diode eine Stromquelle (128) zur Diodenpolarisation umfasst.

3. Empfangsschaltung nach Anspruch 1, bei der die erste nicht kohärente Erfassungsschaltung mit Diode einen Detektor mit zwei Dioden (130, 132) umfasst, die als Spannungsverdoppler ausgeführt sind.

4. Empfangsschaltung nach Anspruch 1, bei der die Frequenz (F_{c}) der Hochfrequenzträgerwelle in dem Band ISM 2,4 GHz oder in dem Band RFID 900 MHz enthalten ist.

5. Empfangsschaltung nach Anspruch 1, bei der die Frequenz (Fₘ) der Niederfrequenzträgerwelle zwischen 25 und 500 kHz beträgt.

6. Empfangsschaltung nach Anspruch 1, bei der die Frequenz (D_{b}) des Signals im Basisband zwischen 5 und 100 kbps beträgt.

7. Empfangsschaltung nach Anspruch 1, bei der die Verstärkung des aktiven Bandpassfilters (122) zwischen 30 und 50 dB beträgt.

## Claims

1. A RF telemetry receiver circuit for active medical implant, with a reception chain comprising successively:
- an antenna (104) for reception of a RF signal;
- an upstream stage (108) for pass-band filtering and impedance matching of the signal picked-up by the antenna;
- a stage for detecting the envelope of the signal delivered by the upstream filtering stage; and
- a stage (116) for digital demodulation of the signal delivered by the envelope detection stage,
the envelope detection stage being a stage adapted to demodulate a waveform of a double-modulation RF signal, having:
. a first modulation, of a first carrier (*b*) by a binary signal (*a*) in baseband (D_{b}) to be transmitted, to obtain a first modulated signal (*d*), and
. a second modulation, to obtain a second modulated signal (*e*) to be transmitted,
**characterized in that**:
- the second modulation is a modulation of a second carrier (*c*) by the first modulated signal, the first carrier being low-frequency (Fₘ) and the second carrier being high-frequency (F_{c});
- the upstream filtering stage (108) is centered on the frequency (Fc) of the high-frequency carrier; and
- the envelope detection stage is a non-heterodyne detection stage, devoid of local oscillator and mixer, and comprises:
. a first diode-based non-coherent detection circuit (120), receiving as an input the signal delivered by the upstream filtering stage;
. an active pass-band filter (122), receiving as an input the signal (*f*) delivered by the first diode-based non-coherent detection circuit, this active pass-band filter being centered on a frequency (2.Fₘ) twice that (Fₘ) of the low-frequency carrier and having a bandwidth (2.D_{b}) twice that of the baseband (D_{b}); and
. a second diode-based non-coherent detection circuit (124), receiving as an input the signal (*g*) delivered by the active pass-band filter and delivering as an output a baseband signal (*h*) applied to the digital demodulation stage (116).

2. The receiver circuit of claim 1, wherein the first diode-based non-coherent detection circuit comprises a source (128) of diode-polarisation current.

3. The receiver circuit of claim 1, wherein the first diode-based non-coherent detection circuit comprises a detector with two diodes (130, 132) configured as a voltage doubler.

4. The receiver circuit of claim 1, wherein the frequency (F_{c}) of the high-frequency carrier is comprised in the ISM band 2.4 GHz or in the RFID band 900 MHz.

5. The receiver circuit of claim 1, wherein the frequency (Fₘ) of the low-frequency carrier is comprised between 25 and 500 kHz.

6. The receiver circuit of claim 1, wherein the frequency (D_{b}) of the baseband signal is comprised between 5 and 100 kbps.

7. The receiver circuit of claim 1, wherein the gain of the active pass-band filter (122) is comprised between 30 and 50 dB.
